# EUROPEAN PATENT APPLICATION

(11) **EP 4 470 477 A1**
(43) Date of publication of application: **04.12.2024**
(21) Application number: 23176023.2
(22) Date of filing: 30.05.2023
(51) Int. Cl.: A61B 10/02

(54) **COMPOSITE NEEDLE ARCHITECTURE**

(71) Applicant: Xaga Surgical AB, 256 67 Helsingborg (SE)
(72) Inventor: FORSVALL, Andreas, 256 67 HELSINGBORG (SE)
(74) Representative: Brann AB

(57) **Abstract**

A needle arrangement (10) intended for medical procedures, wherein the needle arrangement (10) comprises: a sheath (12), a shaft (14), and a head (16). The sheath (12) is elongated and forms an elongated tube having a proximal opening (18) and a distal opening (20), the head (16) is connected to the shaft (14), and the shaft (14) extends through the sheath (12). The head (16) has a closed position relative to the sheath (12) in which the head (16) contacts the sheath (12) at the distal opening. The head (16) also has open position relative to the sheath (12) in which the head (16) is spaced apart from the sheath (12), and the head (16) is of plastic.

## Description

### Technical field

The proposed technology relates generally to the field of biopsy needles and injection/aspiration needles.

### Background

It is known to manufacture biopsy needles and injection/aspiration needles of steel. Biopsy needles typically have a needle sheath with a needle shaft extending within the needle sheath. New biopsy needles have been developed in which a head is attached to the needle shaft. The head may be subjected high impacts from the sheath when taking biopsy samples. However, the manufacturing of a shaft with a head is costly in terms of time and labor, particularly since the head must be secured such that it does not come off during use.

### Object

The proposed technology aims at making the manufacturing of biopsy needles and injection/aspiration needles having a head securely attached to the needle shaft easier. A further object of the proposed technology is to provide a biopsy needle and injection/aspiration needle with plastic components.

### Summary

According to a first aspect of the proposed technology, a needle arrangement is provided. The needle arrangement comprises: a sheath, or needle sheath, a shaft, or needle shaft, and a head, or needle tip. The sheath is elongated and forms, or constitutes, an elongated tube having a proximal opening and a distal opening. The head is attached, or connected, to the shaft and the shaft extends through the sheath. The head has a closed position relative to the sheath in which the head engages, or contacts, the sheath at the distal opening. Worded differently, the head may be located at the distal opening in the closed position of the head. The head has open position relative to the sheath in which the head is spaced apart from the sheath, or the distal opening of the sheath, and the head is of or at least partly of plastic, or the head is manufactured from or at least partly manufactured from plastic. In comparison with a head of steel, this allows for less demanding methods of attaching the head to the sheath.

The head may form a tip or cutting edge for penetrating tissue. The tip or the cutting edge may be honed or sharpened by honing or grinding.

It is understood that the needle arrangement is intended for medical procedures, such as percutaneous procedures. It is further understood that the shaft is accessible at the proximal opening, both in the closed position and in the open position of the head. It is further understood that the shaft extends along the sheath. It is further understood that the shaft extends within the tube formed by the sheath. It is further understood that the shaft is arranged to, or can, slide relative to the sheath. It is further understood that the sheath and the shaft are elongated.

It is specified that the head is attached, or connected, to the shaft. This encompasses the head being fused to, or integrated with, the shaft.

The elongated tube formed by the sheath may have an internal, or inner, transverse extension, or diameter. The head may have an external, or outer, transverse extension, or diameter that is greater than the internal transverse extension of the elongated tube. The transverse extension is understood to be relative to the sheath. This means that the head is prevented from entering the sheath.

The sheath and the head may jointly form an outer, or external, surface at the distal opening in the closed position of the head. The sheath and the head may jointly form an intersection, or seam, in the outer surface. The outer surface may have a cylindrical geometry, such as a circular cylindrical geometry. The outer surface may be smooth. For example, the head contact may contact the sheath along the complete intersection at the outer surface. The head may seal to the sheath in the closed position.

It is specified above that the head is of, or at least partly of, plastic. The shaft may be of plastic or manufactured from plastic. Alternatively, the shaft may be of metal, such as stainless steel. The sheath may be of plastic or manufactured from plastic. Alternatively, the sheath may be of metal, such as stainless steel.

The complete needle arrangement may be of plastic, which for example allows it to be used simultaneous to magnetic resonance imaging. It is understood that the head, shaft, and sheath may be of different types of plastics.

The plastic of the head, the shaft and/or the sheath may be of, or manufactured from, PolyEthylene (PE), such as High-Density PolyEthylene (HDPE) and Ultra-High-Molecular-Weight PolyEthylene (UHMWPE). Alternatively, the head, the shaft, and or the sheath may be of, or manufactured from, PolyEther Ether Ketone (PEEK). These materials have high impact strength, in particular UHMWPE and PEEK. This allows for the needle arrangement to close, or the head to transition from the open position to the closed position, with a grater force without the needle arrangement breaking at the head. The plastic may be fiber reinforced, for example by glass fibers or carbon fibers.

The head may be attached to the shaft by a screw fit. This contributes to a secure attachment of the head. Alternatively, the head may be fused to the shaft, for example by heating.

The complete head may be of plastic. This means that the abovementioned tip or cutting edge is defined by the plastic.

Alternatively, the head may be a composite head. The head may comprise a first part and a second part, wherein the first part is of plastic and the second part is of metal. The first part and the second part are joined to form a cohesive structure. For example, the metal part may be of metal, such as stainless steel. It is understood that that the first part and the sheath may jointly form the abovementioned outer surface at the distal opening.

The first part may be attached, or connected, to the shaft. The second part may form the tip or cutting edge, or at least a part of the tip or cutting edge. The first part and the second part may form matching interlocking structures. It is understood that the interlocking structures are arranged to prevent the first part and the second part from separating. The interlocking structures may be internal to the head, which means that they cannot be accessed from outside the head. The first part and the second part may be juxtaposed or form a continuous structure. This means that there is no gap between the first part and the second part, which contributes to prevent bacteria or matter from collecting between the first part and the second part.

The composite head has the advantage that a plastic less suitable for forming a tip or cutting edge and more suitable for injection molding can be used.

The head, or the first part of the head, and the shaft may form a monolithic structure. This means that two components are seamlessly joined to one another and that the components have not been manufactured separately and later assembled. It is understood that head and the shaft may be of the same plastic. The monolithic structure reduces the risk of the head coming loose or breaking off at a transition from the open position to the closed position.

The complete head and the shaft may have been jointly, or simultaneously, formed, or manufactured, by a single injection molding. Preferably, the head and the shaft are of injection molded UHMWPE.

Alternatively, the first part of the head and the shaft may have been jointly, or simultaneously, formed, or manufactured, by a single injection molding. Preferably, the first part and the shaft are of injection molded HDPE. The second part may have been present in the injection molding to form the abovementioned cohesive structure. For example, the second part may have been positioned in the mold before the injection molding.

As mentioned above, the injection molding contributes to reduce the risk of the head coming off. The tip or the cutting edge may be honed or sharpened by honing or grinding. It is understood that this may apply both when the complete head is of plastic and when it has a second part of metal.

The head and the shaft may be a combined structure. This means that the head and the shaft have been manufactured separately and later combined. The complete head may be of plastic, as described above. The head may then have been formed, or manufactured, by injection molding. Alternatively, the head may have a first part and a second part, as described above. The second part may then have been formed, or manufactured, by injection molding with the second part present in the injection molding to form the abovementioned cohesive structure. For example, the second part may have been positioned in the mold before the injection molding.

The head, or the first part of the head, may have, or form, a first mechanical interface and the shaft may have, or form, a second mechanical interface that cooperates with the first mechanical interface and attaches the head to the shaft. For example, the first interface may be a female threading and the second interface may be male threading. The first mechanical interface may be formed in the abovementioned injection molding.

It is specified above that the shaft may be of plastic. The head, or the first part of the head, and the shaft may then be fused together, for example by heating.

The needle arrangement may be a biopsy needle. The sheath and the shaft may jointly form a cavity with the head in the closed position for holding a biopsy sample. The cavity may be exposed from, or located outside, the sheath with the head in the open position.

The shaft may have, or form, a cutout that forms the cavity together with the sheath. The cutout may face a first direction and the head may be configured to be biased in an opposite second direction in a transition from the closed position to the open position at a passage through tissue. The cutout may cause the shaft to bend in the first direction and the biasing will compensate for this. Worded differently, the sheath may define a cylindrical geometry having a cylinder axis, wherein the cutout is facing a first direction relative to the cylinder axis and the head has a tip that is offset relative to the cylinder axis in an opposite second direction. This offset will bias the head as described above. The biasing is particularly advantageous if the shaft is of plastic since such materials typically has a lower stiffness than steel.

Alternatively to the shaft having a cutout that forms the cavity together with the sheath, the needle arrangement may further comprise an auxiliary shaft that extends within the tube formed by the sheath and is arranged to, or can, slide relative to the sheath and the shaft. The auxiliary shaft may have a forward position, or closed position, relative to the head and a rear position, or open position, relative to the head. The auxiliary shaft may have a proximal end and a distal end, and the distal end may be located at the head in the forward position of the auxiliary shaft. The distal end may be spaced apart from the head in the rear position of the auxiliary shaft.

It is understood that the auxiliary shaft is elongated. It is further understood that the shaft and the auxiliary shaft are parallel and that the shaft extends along the shaft, or along the sheath. With the distal end of the auxiliary shaft located at the head, or in the forward position of the auxiliary shaft, the auxiliary shaft may be accessible at the proximal opening of the elongated tube formed by the sheath, both in the closed position and in the open position of the head. In other words, the auxiliary shaft extends outward from the proximal opening in the open position of the head and in the forward position of the auxiliary shaft.

The sheath and the shaft may jointly form a cavity for holding a biopsy sample with the head in the closed position and the auxiliary shaft in the rear position. The cavity may be exposed for receiving a biopsy sample with the head in the open position and the auxiliary shaft in the rear position. The distal end of the auxiliary shaft may be located between the sheath and the head in the open position of the head and in the rear position of the auxiliary shaft. Alternatively, the distal end of the auxiliary shaft may be located within the elongated tube formed by the sheath in the open position of the head and in the rear position of the auxiliary shaft The cavity may be blocked from receiving a biopsy sample by the auxiliary shaft with the head in the open position and the auxiliary shaft in the forward position. The rear position of the auxiliary shaft may be variable. This means that the length of the cavity is variable and that biopsies of different lengths can be made.

The auxiliary shaft may be arranged to support the head and/or the shaft in the forward position of the auxiliary shaft, for example in the open position of the head and/or in a transition from the closes position to the open position of the head. For example, the auxiliary shaft, or the distal end of the auxiliary shaft, may engage, or contact, the head, or a part of the shaft at the head, in the forward position. This contributes to prevent the shaft from bending towards the auxiliary shaft in a transition from the closed position to the open position of the head. This is advantageous if the shaft is of plastic, which typically has a lower stiffness than stainless steel.

The head, or a part of the shaft at the head, may form a releasable lock, or catch, arranged to prevent the distal end of the auxiliary shaft from moving sideways, or transverse, relative to the shaft in the forward position of the auxiliary shaft. For example, the head may form an indentation, or depression, and the distal end of the auxiliary shaft is seated in the indentation in the forward position. This contributes to prevent the shaft from bending away from the auxiliary shaft, or vice versa, in a transition from the closed position to the open position of the head.

The auxiliary shaft may be of plastic, for example any of the types of plastic specified above. Alternatively, it may be of metal, such as stainless steel.

The sheath may form a cutting edge for cutting a biopsy sample from tissue. The cutting edge of the sheath may pass over the abovementioned cutout in a transition from the open position to the closed position of the head. The cutting edge of the sheath may be honed or sharpened by honing or grinding.

The needle arrangement may be a fluid needle, such as an injection and/or aspiration needle. The tube formed by the sheath may form, or constitute, a cannula, or conduit, extending between the proximal opening to the distal opening for transferring a liquid. The shaft may be configured to allow passage of a liquid through the tube formed by the sheath with the head in the open position. It is understood that the liquid can enter/exit the cannula via the proximal opening to the distal opening. Worded differently, the sheath and the shaft may form the cannula between them that extends between the proximal opening to the distal opening. Alternatively, the shaft may form a cannula, or conduit, having a first inlet/outlet and a second inlet/outlet, wherein the sheath is positioned between the first inlet/outlet and the second inlet/outlet with the head in the open position. The second inlet/outlet may be located at the head. The first inlet/outlet may be located at the opposite end of the shaft relative to the head.

According to a second aspect of the proposed technology, a method for manufacturing a shaft and a head for a needle assembly is proposed. The method comprises: providing a shaft having a male threading, injection molding a head of plastic having a cooperating female threading, and mating the male threading and the female threading. The shaft may be of metal, such as stainless steel, or the shaft may be provided by injection molding the shaft of plastic.

Alternatively, the method comprises: injection molding a shaft of plastic, injection molding a head of plastic, and fusing the shaft and the head together, for example by heating. Alternatively, the method comprises: injection molding a shaft and a head of plastic simultaneously, or jointly, in a single mold.

Alternatively, the method comprises: providing a head having a first mechanical interface, providing a shaft having a second mechanical interface arranged to cooperate with the first mechanical interface, and mating the first mechanical interface and the second mechanical interface. The shaft may be of metal, such as stainless steel, or the shaft may be provided by injection molding the shaft of plastic. Providing the head may then comprise: injection molding the complete head of plastic. Alternatively, providing the head may comprise: placing a second part of a head in a mold and injection molding a first part of the head in the mold to form a head having a cohesive structure. The first part and the second part may have any of the features described above.

Alternatively, the method comprises: placing second part of a head in a mold and injection molding a first part of the head and the shaft in the mold to form a head having a cohesive structure. The first part and the second part may have any of the features described above.

The abovementioned methods may further comprise: honing or grinding the head, or the second part of the head, to form a tip or cutting edge for penetrating tissue. Alternatively, the head may have a tip or cutting edge that is provided, or formed, in the injection molding. The abovementioned methods may further comprise: providing a sheath, and positioning the shaft in a sheath. The sheath, the shaft, and the head may have any of the features described above in relation to the first aspect of the proposed technology.

### Brief description of the drawings

A more complete understanding of the abovementioned and other features and advantages of the proposed technology will be apparent from the following detailed description in conjunction with the appended drawings, wherein:
- Figures 1: is side views of a biopsy needle with the head in an open position,
- Figures 2a and 2b: are side views of the front part of the biopsy needle in Figure 1 with the head in a closed position and an open position, respectively,
- Figures 3a and 3b: are cross sectional views of the front part of the biopsy needle in Figure 1 with the head in a closed position and an open position, respectively,
- Figures 4a and 4b: are cross sectional views of the front part of an alternative biopsy needle with the head in a closed position and an open position, respectively,
- Figures 5a and 5b: are side views of the front part of an injection and/or aspiration needle with the head in a closed position and an open position, respectively,
- Figures 6a and 6b: are cross sectional views of the front part of the injection and/or aspiration needle in Figures 5a and 5b with the head in a closed position and an open position, respectively,
- Figures 7a and 7b: are cross sectional views of the front part of an alternative injection and/or aspiration needle with the head in a closed position and an open position, respectively,
- Figures 8a and 8b: are a side view respective a cross sectional view of the front part of an alternative biopsy needle having a composite head,
- Figures 9a and 9b: are a side view respective a cross sectional view of the front part of another alternative biopsy needle having a composite head,
- Figures 10a to 10d: are cross sectional views of the front part of another alternative biopsy needle having an auxiliary shaft, and
- Figure 11: is cross sectional views of the front part of another alternative biopsy needle having an auxiliary shaft.

### Description of the drawings

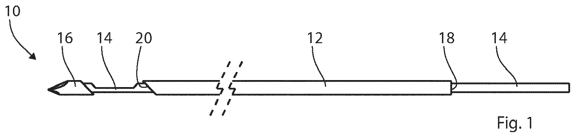

Figures 1, 2a, 2b, 3a, and 3b illustrates an embodiment of a biopsy needle 10. The biopsy needle 10 has an elongated sheath 12 forming a tube having a proximal opening 18 and a distal opening 20. The biopsy needle 10 also has a shaft 14 that extends within through the sheath 12 and a head 16 is attached connected to the shaft 14. The head 16 has a closed position relative to the sheath 12 in which the head 16 contacts and seals to sheath 12 at the distal opening 20 and an open position relative to the sheath 12 in which the head 16 is spaced apart from the sheath 12. The shaft 14 is accessible at the proximal opening 18 both in the closed position and in the open position of the head 16.

The elongated tube formed by the sheath 12 has an internal diameter and the head 16 has an external diameter that is greater than the internal diameter of the elongated tube. The sheath 12 and the head 16 jointly form a circular-cylindrical outer surface 22 at the distal opening 20 in the closed position of the head 16. The sheath 12 and the head 16 also jointly form an intersection 24 in the outer surface 22.

The shaft 14 has a cutout 26 that forms a cavity 26 together with the sheath 12 with the head 16 in the closed position. The cavity 26 is intended to hold a biopsy sample. The cavity 26 is exposed from the sheath 12 with the head 16 in the open position.

The sheath 12 defines a cylindrical geometry having a cylinder axis 28, wherein the cutout 26 is facing upward relative to the cylinder axis 28 and the head 16 has a tip 30 that is offset downward relative to the cylinder axis 28. The head 16 forms a cutting edge 32 for penetrating tissue. the sheath 12 forms a cutting edge 34 for cutting a tissue sample in a forward movement of the sheath 12 relative to the shaft 14 and the head 16. The cutting edge 34 of the sheath 12 passes over the cutout 26 and positions the tissue sample in the cavity 26.

In the embodiment of Figures 1, 2a, 2b, 3a, and 3b, the sheath 12 and the shaft 14 are of stainless steel. The head 16 is of injection molded UHMWPE and formed with a female threading 36. The shaft 14 has a cooperating male threading 36 by which the head 16 is attached. This way, the shaft 14 and the head forms a combined structure of components that have been manufactured separately from one another. In an alternative embodiment, the sheath 12 is of stainless steel and the shaft 14 is of injection molded UHMWPE and formed with the male threading 36. In another alternative embodiment, the sheath 12 is of injection molded UHMWPE and the shaft 14 is of stainless steel and forms the male threading 36. In another alternative embodiment the sheath 12, shaft 14, and the head 16 are of injection molded HDPE, and the head 16 is fused to the shaft 14.

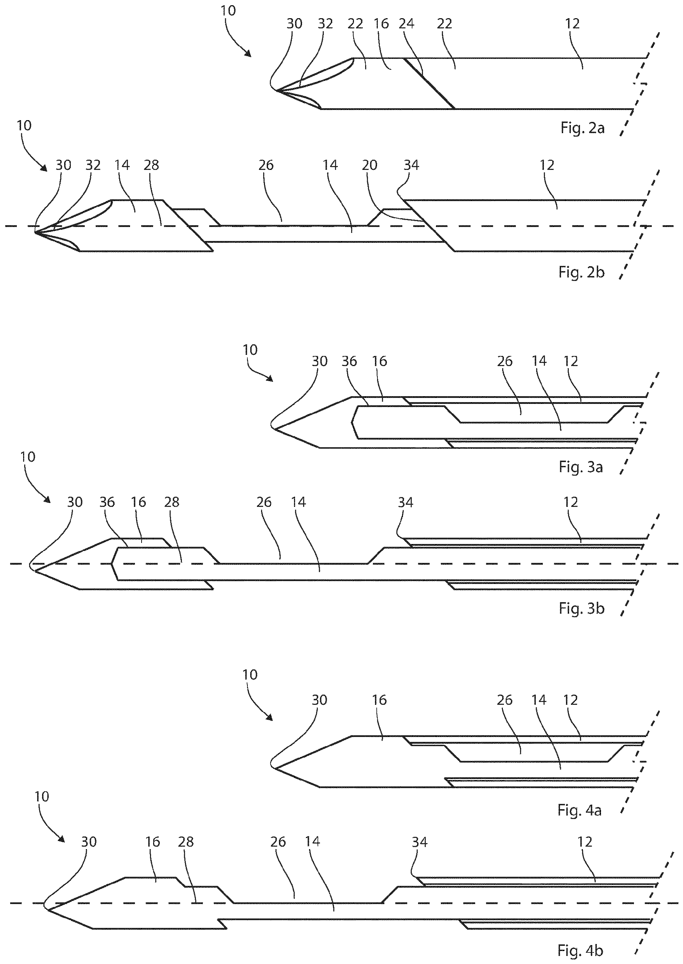

An alternative embodiment of a biopsy needle is shown in Figures 4a and 4b. This embodiment differs from the embodiment of Figures 1, 2a, 2b, 3a, and 3b in that the head 16 and the shaft 14 have been jointly formed in a single injection molding with UHMWPE. It also differs in that the sheath 12 is of injection molded UHMWPE.

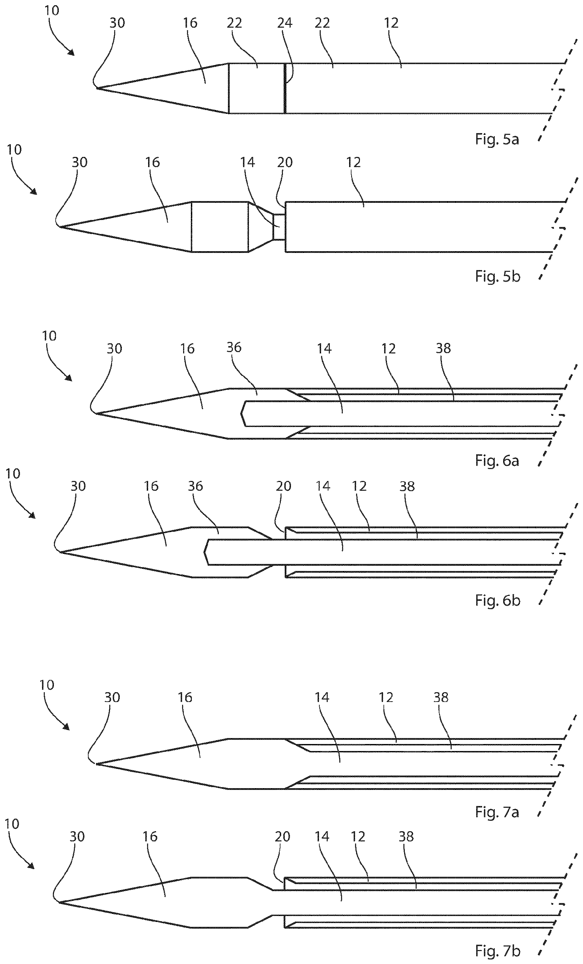

Figures 5a, 5b, 6a, and 6b illustrates an embodiment of an injection/aspiration needle 10. The injection/aspiration needle 10 has an elongated sheath 12 forming a tube having a proximal opening 18 and a distal opening 20. The injection/aspiration needle 10 also has a shaft 14 that extends within and through the sheath 12. A head 16 is attached connected to the shaft 14. The head 16 has a closed position relative to the sheath 12 in which the head 16 contacts and seals to sheath 12 at the distal opening 20 and an open position relative to the sheath 12 in which the head 16 is spaced apart from the sheath 12. The shaft 14 is accessible at the proximal opening 18, both in the closed position and in the open position of the head 16.

The elongated tube formed by the sheath 12 has an internal diameter and the head 16 has an external diameter that is greater than the internal diameter of the elongated tube. The sheath 12 and the head 16 jointly form a circular-cylindrical smooth outer surface 22 at the distal opening 20 in the closed position of the head 16. The sheath 12 and the head 16 also jointly form an intersection 24 in the outer surface 22.

The head 16 forms a tip 30 for penetrating tissue. The sheath 12 and the shaft 14 form the cannula 38 between them that extends between the proximal opening 18 to the distal opening 20 for transferring a liquid. A liquid can enter/exit the cannula via the proximal opening 18 and the distal opening 20 and pass through the tube formed by the sheath 12 with the head 16 in the open position.

In the embodiment of Figures 5a, 5b, 6a, and 6b, the sheath 12 and the shaft 14 are of stainless steel. The head 16 is of injection molded UHMWPE and formed with a female threading 36. The shaft 14 has a cooperating male threading 36 by which the head 16 is attached. In an alternative embodiment, the shaft 14 is of injection molded UHMWPE and formed with the male threading 36. In another alternative embodiment the shaft 14 and the head 16 are of injection molded HDPE and the head 16 is fused to the shaft 14.

An alternative embodiment of an injection/aspiration needle 10 is shown in Figures 7a and 7b. This embodiment differs from the embodiment of Figures 5a, 5b, 6a, and 6b in that the head 16 and the shaft 14 have been jointly formed in a single injection molding with UHMWPE. It also differs in that the sheath 12 is of injection molded UHMWPE.

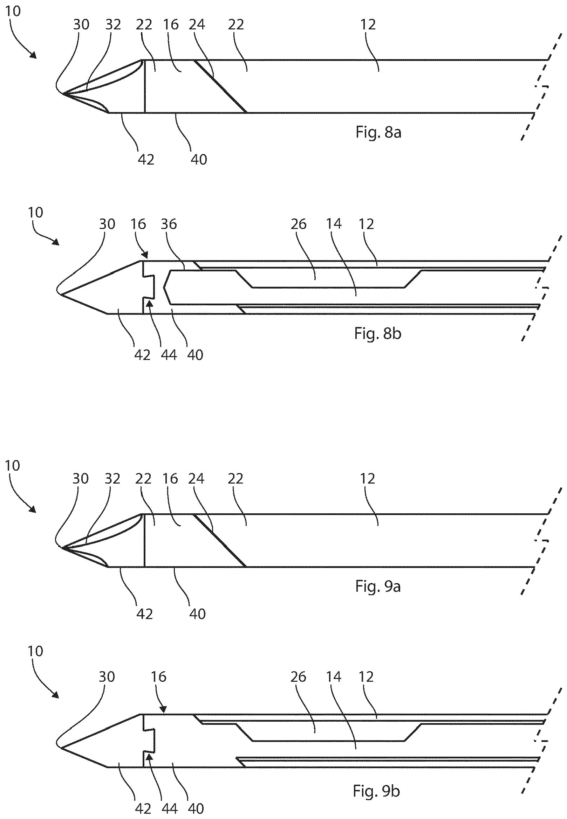

An alternative embodiment of a biopsy needle 10 is shown in Figures 8a and 8b. This embodiment differs from the embodiment described in relation to Figures 1, 2a, 2b, 3a, and 3b by the head 16 having a first part 40 of HDPE and a second part 42 of stainless steel. The first part 40 and the second part 42 forms internal interlocking structures 44 that fixes to the second part 42 to the first part 40, this way forming a cohesive and continuous structure without any gap between the first part 40 and the second part 42. The first part 40 is attached to the shaft 14 and the second part 42 forms the cutting edge 32 of the head 16.

The head 16 has been manufactured by placing the second part 42 of the head 16 in a mold and injection molding UHMWPE in form to form the first part 40. The first part is formed with a female threading 36, and the head 16 is attached to the shaft 14 as described in relation to Figures. 1, 2a, 2b, 3a, and 3b.

An alternative embodiment of a biopsy needle 10 is shown in Figures 9a and 9b. The head 16 shares the features of the head described in relation to Figures 8a and 8b. It differs in that the shaft 14 is of HDPE. The shaft 14 and the head 16 have been manufactured by placing the second part 42 of the head 16 in a mold and injection molding HDPE in the mold to simultaneously form the first part 40 and the shaft 14. This way, the second part 42 of the head and the shaft 14 form a monolithic structure.

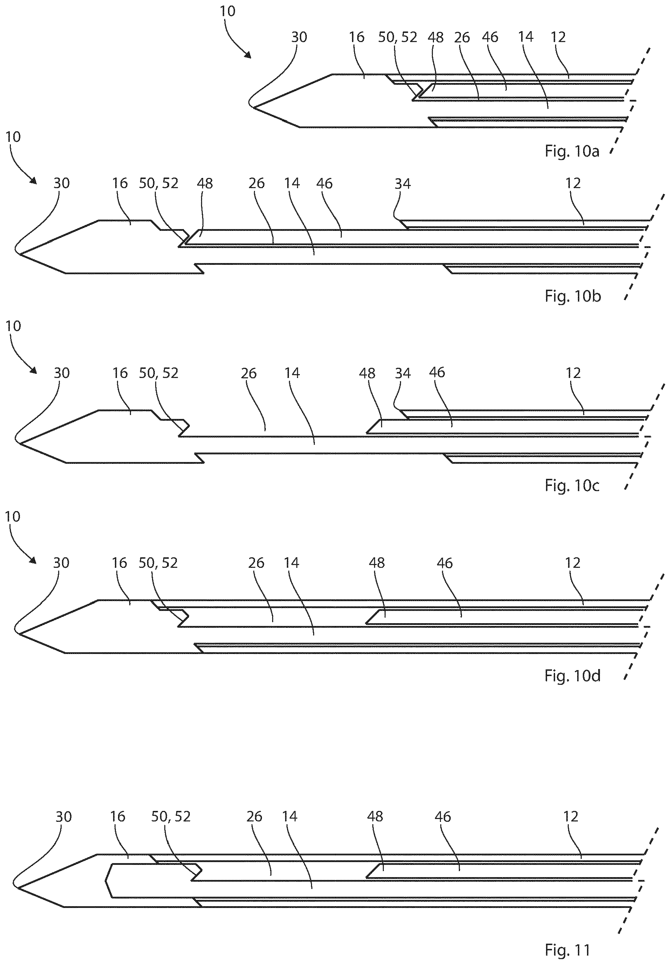

An alternative embodiment of a biopsy needle 10 is shown in Figures 10a to 10d. The biopsy needle 10 has the general features of the embodiment of Figures 4a and 4b and differs by further having an elongated auxiliary shaft 46 that is parallel with the shaft 14 and extends within the tube formed by the sheath 12. The auxiliary shaft can slide relative to the sheath 12 and the shaft 14. It has a forward position and a rear position relative to the head 16. The forward position is shown in Figures 10a and 10b, and the rear position is shown in Figures 10c and 10d. The head 16 is in the closed position in Figures 10a and 10d and in the open position in Figures 10b and 10c. In use, the biopsy needle 10 transitions sequentially from the configuration in Figure 10a to the configuration in Figure 10d, as is further described in WO 2022/207605.

The auxiliary shaft 46 has a proximal end (not shown) and a distal end 48. The distal end 48 is located at the head 16 in the forward position of the auxiliary shaft 46 and is spaced apart from the head 16 in the rear position of the auxiliary shaft 46. The auxiliary shaft 46 is accessible at the proximal opening 18 of the elongated tube formed by the sheath 12 (see Figure 1) in its forward position, both in the closed position and in the open position of the head 16.

The sheath 12 and the shaft 14 jointly form a cavity for holding a biopsy sample when the head 16 is in the closed position and the auxiliary shaft 46 is in the rear position, see Figure 10d. The cavity 26 is exposed and can receive a biopsy sample when the head 16 is in the open position and the auxiliary shaft 46 is in the rear position, see Figure 10c. The cavity 26 is blocked from receiving a biopsy sample by the auxiliary shaft 46 when the head 16 is in the open position and the auxiliary shaft 46 is in the forward position. The auxiliary shaft 46 is held in the forward position in a transition from the closed position to the open position of the head 16. The rear positions of the auxiliary shaft 46 is variable, which allows for biopsies of different lengths.

The distal end 48 of the auxiliary shaft 48 contacts the head 14 in the forward position. The head 16 forms an indentation 50 and the distal end 48 of the auxiliary shaft 46 is seated in the indentation 50, which prevents the distal end 48 from moving sideways relative the shaft 14. This way, the head 16 forms a releasable lock 52 for the distal end 48. Additionally, the auxiliary shaft 46 is arranged to support the head 16 in the forward position of the auxiliary shaft4 6 and contributes to prevent the shaft 14 from bending towards and away from the auxiliary shaft 46 in a transition from the closed position to the open position of the head 16.

The auxiliary shaft 46 is of injection molded UHMWPE. In an alternative embodiment, the auxiliary shaft 46 is of stainless steel.

An alternative embodiment of a biopsy needle 10 is shown in Figure 11. The biopsy needle 10 has the general features of the embodiment of Figures 3a and 3b and differs by further having an elongated auxiliary shaft 46 arranged as described in relation to Figures 11a to 11d. The biopsy needle 10 is depicted in Figure 11 with the head 16 in the closed position and the auxiliary shaft 46 in the rear position. It differs from the embodiment of Figures 11a to 11d by a part of the shaft 14 at the head 16 is forming the releasable lock 52 instead of the head 16.

### Item list

- 10: needle arrangement, biopsy needle, or inj./asp. needle
- 12: sheath
- 14: shaft
- 16: head
- 18: proximal opening
- 20: distal opening
- 22: outer surface
- 24: intersection
- 26: cavity or cutout
- 28: cylinder axis
- 30: tip of head
- 32: cutting edge of head
- 34: cutting edge of sheath
- 36: threading
- 38: cannula
- 40: first part of head
- 42: second part of head
- 44: interlocking structures
- 46: auxiliary shaft
- 48: distal end
- 50: indentation
- 52: lock

## Claims

1. A needle arrangement (10) intended for medical procedures, wherein the needle arrangement (10) comprises:
- a sheath (12),
- a shaft (14),
- a head (16),
wherein
the sheath (12) is elongated and forms an elongated tube having a proximal opening (18) and a distal opening (20), the head (16) is connected to the shaft (14), the shaft (14) extends through the sheath (12), the head (16) has a closed position relative to the sheath (12) in which the head (16) contacts the sheath (12) at the distal opening, the head (16) has open position relative to the sheath (12) in which the head (16) is spaced apart from the sheath (12), and the head (16) is of plastic or at least partly of plastic.

2. The needle arrangement (10) according to claim 1, wherein the elongated tube formed by the sheath (12) has an internal transverse extension, and the head (16) has an external transverse extension that is greater than the internal transverse extension of the elongated tube.

3. The needle arrangement (10) according to claim 1 or 2, wherein the sheath (12) and the head (16) jointly form an outer surface (22) at the distal opening (20), the sheath (12) and the head (16) jointly form an intersection (24) in the outer surface (22), and the outer surface (22) has a cylindrical geometry.

4. The needle arrangement (10) according to any of the claims 1 to 3, wherein the shaft (14) is of plastic.

5. The needle arrangement (10) according to any of the claims 1 to 4, wherein the plastic of the head (16) and/or the shaft (14) is of High-Density PolyEthylene (HDPE) or Ultra-High-Molecular-Weight PolyEthylene (UHMWPE).

6. The needle arrangement (10) according to any of the claims 1 to 5, wherein the head (16) is attached to the shaft (14) by a screw fit.

7. The needle arrangement (10) according to any of the claims 1 to 6, wherein the head (16) and the shaft (14) form a monolithic structure.

8. The needle arrangement (10) according to any of the claims 1 to 7, wherein the head (16) and the shaft (14) have been jointly formed by a single injection molding.

9. The needle arrangement (10) according to any of the claims 1 to 8, wherein the needle arrangement (10) is a biopsy needle (10), the sheath (12) and the shaft (14) jointly form a cavity (26) with the head (16) in the closed position for holding a biopsy sample, and the cavity (26) is exposed from the sheath (12) with the head (16) in the open position.

10. The needle arrangement (10) according to claim 9, wherein the shaft (14) has a cutout (26) that forms the cavity (26) together with the sheath (12), the cutout (26) faces a first direction and the head (16) is configured to be biased in an opposite second direction in a transition from the closed position to the open position at a passage through tissue.

11. The needle arrangement (10) according to any of the claims 1 to 8, wherein the needle arrangement (10) is a biopsy needle (10), and the needle arrangement (10) further comprises:
- an auxiliary shaft (46) that extends within the tube formed by the sheath (12) and is arranged to slide relative to the sheath (12) and the shaft (14),
wherein the auxiliary shaft (46) has a forward position and a rear position relative to the head (16), the auxiliary shaft (46) has a proximal end and a distal end (48), and the distal end (48) is located at the head (16) in the forward position of the auxiliary shaft (46) and spaced apart from the head (16) in the rear position of the auxiliary shaft (46).

12. The needle arrangement (10) according to claim 11, wherein the auxiliary shaft (46) is arranged to support the head (16) and/or the shaft (14) in the forward position of the auxiliary shaft (46).

13. The needle arrangement (10) according to any of the claims 1 to 8, wherein the needle arrangement (10) is an injection and/or aspiration needle (10), the tube formed by the sheath (12) forms a cannula (38) extending between the proximal opening (18) to the distal opening (20) for transferring a liquid.

14. A method for method for manufacturing a shaft (14) and a head (16) for a needle assembly (10), wherein the method comprises:
- providing a shaft having a male threading, injection molding a head of plastic having a cooperating female threading (36), and mating the male threading and the female threading (36), wherein the shaft (14) is of steel; or
- injection molding a shaft (14) of plastic; injection molding a head (16) of plastic, and fusing the shaft and the head together; or
- injection molding a shaft (14) and a head (16) of plastic simultaneously in a single mold.

15. The method according to claim 14, wherein the method further comprises:
- honing or grinding the head to form a tip or cutting edge for penetrating tissue.
